(19)

Europäisches
Patentamt

European
Patent Office

Office européen
des brevets

(11) **EP 3 214 441 A1**

(12) **EUROPEAN PATENT APPLICATION**

(43) Date of publication:
**06.09.2017 Bulletin 2017/36**

(51) Int Cl.:
***G01N 33/50*** *(2006.01)*

(21) Application number: **16158265.5**

(22) Date of filing: **02.03.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Rheinisch-Westfälische Technische
Universität
Hochschule (RWTH) Aachen
52062 Aachen (DE)**

(72) Inventors:
• **Schilling, Jana
52074 Aachen (DE)**

• **Büchs, Jochen
52064 Aachen (DE)**
• **Nolten, Jannis
72070 Tübingen (DE)**
• **Schulte, Andreas
52062 Aachen (DE)**
• **Conrath, Uwe
4720 Kelmis (BE)**
• **Schillheim, Britta
52134 Herzogenrath (DE)**

(74) Representative: **Lasar, Andrea Gisela
Maiwald Patentanwalts GmbH
Elisenhof
Elisenstraße 3
80335 München (DE)**

(54) **METHOD FOR IDENTIFYING SUBSTANCES WHICH PRIME A DEFENSE RESPONSE**

(57)    The present invention relates to a method for identifying substances which prime cells for a stress response by measuring the production of at least one volatile organic compound of the cells treated with a candidate substance in comparison to cells not treated with the candidate substance.

EP 3 214 441 A1

**Description**

**FIELD OF THE INVENTION**

The present invention relates to a method for identifying substances which prime cells for a stress response by measuring the production of at least one volatile organic compound of the cells treated with a candidate substance in comparison to cells not treated with the candidate substance as well as a device suitable for use in said method.

**BACKGROUND OF THE INVENTION**

Plant diseases which are caused by various pathogens such as viruses, bacteria, oomycetes, nematodes and fungi or abiotic stress such as drought, cold, freeze and salt may lead to significant crop losses of cultivated plants, resulting in economic detriments and in threatening food and feed supply.

Since the last century, chemical pesticides have been used for controlling plant diseases. Nevertheless, at present at least 40% of the possible plant yield is lost due to diseases and abiotic stress such as drought (Oerke et al. (1994) Crop production and crop protection, Elsevier Science B.V, ISBN 0 444 82095 7). Hence, there is still a need for improved methods for controlling plant diseases and abiotic stresses. Due to the low acceptance of genetically modified plants in Europe and because of the increasing demand for substances which are uncritical to the environment, there is a huge interest in using natural or near-natural substances for an effective plant protection.

Natural or near-natural substances are particularly effective, if they stimulate certain parts of the plant's endogenous immune system. This is particularly true for agents which act via the so-called "defense priming" (reviewed in Conrath et al. (2015) Annual Review of Phytopathology 53: 97-119; Conrath (2011) Trends in Plant Science 16(10): 524-531; Conrath et al. (2006) Mol. Plant. Microbe Interact. 19(10): 1062-1071). Defense priming prepares the plant's endogenous immune system for a future challenge such as pathogen infection or abiotic stress, but does not directly activate immunity. However, primed plants respond to very low levels of a stimulus, such as biotic or abiotic stress, in a more rapid and robust manner than unprimed plants. Hence, substances which induce defense priming confer an increased stress tolerance and disease resistance to plants without reducing the yield. Consequently, there is a strong demand for substances which induce defense priming in plants.

It has been shown that defense priming is not restricted to plant cells, but also occurs in animal cells (see, e.g., Hayes et al. (1991) J. Leukocyte Biol. 50: 176-181). For example, interferon-y or GM-CSF can prime the induction of the expression of the cytokines interferon-$\alpha$, interferon-$\beta$, tumour necrosis factor $\alpha$ and IL-12 which cytokines are involved in the overall defense response (Hayes et al. (1995) Blood 86: 646-650).

However, since the defense priming process does not lead to a direct activation of the defense mechanisms, the search for substances which act via defense priming is rather difficult. In parsley cells, it is possible to identify defense priming substances by measuring the enhanced fluorescence after priming with these substances and subsequent treatment with a microbe-associated signal (Siegrist et al. (1998) Physiol. Mol. Plant Pathol. 53(4): 223-238). However, this system cannot be used with other, economically more important, plant species or animal cells. Further, this system does not allow the continuous monitoring of the defense response, as the fluorescence is measured only at one specific timepoint.

International patent application PCT/EP2015/069593 and Schilling et al. (2015) BMC Plant Biology 15: 282 disclose a method for identifying substances which prime cells or organisms for a defense response by determining the respiration activity of the cells treated with the substance in comparison to the respiratory activity in control cells.

Nevertheless, there is still a need for a generally applicable method which enables the identification of substances which prime cells or organisms for a defense response. Additionally, there is a need for a method which allows the investigation of the molecular basis of defense priming.

**OBJECT AND SUMMARY OF THE INVENTION**

It is, thus, an object of the present invention to provide a method for identifying substances which prime cells or organisms for a stress response. It is also an object of the present invention to provide a method for identifying the pathways involved in defense priming by a specific substance.

This and further objects of the invention, as will become apparent from the description, are attained by the subject-matter of the independent claims.

Some of the preferred embodiments of the present invention form the subject-matter of the dependent claims.

The present inventors have surprisingly found that after treatment of cells with a substance which is known to prime cells for a stress response the production of volatile organic compounds such as ethylene and nitric oxide by the cells increases. The increased production of volatile organic compounds such as ethylene and/or nitric oxide can be detected by methods and apparatuses such as the <u>r</u>espiration <u>a</u>ctivity <u>mo</u>nitoring <u>s</u>ystem (RAMOS) which, however, is modified for the detection of volatile organic compounds (so-called modRAMOS).

The above finding can be used to identify further substances which prime cells for a stress response by measuring the production and release of at least one volatile organic compound such as ethylene or nitric oxide by the cells. The method of the present invention can be used with any interesting cell type and the substances identified can then be directly used in combination with this cell type. Further, as the method of the present invention involves the continuous monitoring of the volatile organic compound, more information on the interaction of the candidate substance with the cell can be obtained. By measuring the production of different volatile organic compounds information on the pathways involved in priming by a specific substance can be obtained. The present invention has the advantage that the production of the at least one volatile organic compound can be measured in intact cells and under physiological conditions.

Accordingly, the present invention provides a method for identifying substances which prime cells for a stress response comprising the steps of:

- inoculating cells in a suitable culture medium and culturing them in said medium;
- adding one or more candidate substances to the culture medium thereby treating the cells with said candidate substance; and
- measuring the production of at least one volatile organic compound in the cells treated with the candidate substance and in control cells not treated with the candidate substance,

wherein an increase in the production of the at least one volatile organic compound in the cells treated with the candidate substance compared to the control cells indicates that the candidate substance primes the cells for a stress response.

Preferably, the at least one volatile organic compound is selected from the group consisting of ethylene, nitric oxide and hydrogen peroxide.

The production of the at least one volatile organic compound may be measured in a shaken measuring flask under sterile conditions, wherein during a purge phase air passes through the measuring flask and in a measuring phase the air stream is interrupted and the concentration of the at least one volatile organic compound is measured with a detecting element for said at least one volatile organic compound.

The detecting element may be calibrated, preferably by passing a defined flow rate of the at least one volatile organic compound through the measuring flask during the purge phase and measuring the concentration of the at least one volatile organic compound with the detecting element.

The present invention also related to a method for identifying substances which prime plant cells for a stress response comprising the steps of:

- inoculating plant cells in a suitable culture medium and culturing them in said medium;
- adding one or more candidate substances to the culture medium thereby treating the plant cells with said candidate substance; and
- measuring the production of ethylene in the cells treated with the candidate substance and in control cells not treated with the candidate substance,

wherein an increase in the production of ethylene in the cells treated with the candidate substance compared to the control cells indicates that the candidate substance primes the plant cells for a stress response.

The production of ethylene may be measured in a shaken measuring flask under sterile conditions, wherein during a purge phase air passes through the measuring flask and in a measuring phase the air stream is interrupted and the concentration of ethylene is measured with a detecting element for ethylene.

The detecting element may be calibrated, preferably by passing a defined flow rate of ethylene through the measuring flask during the purge phase and measuring the concentration of ethylene with the detecting element.

The candidate substance may be added 24 to 120 hours after inoculation.

After the addition of the candidate substance, preferably 6 to 48 hours after addition of the candidate substance, the cells may be subjected to a compound eliciting a stress response.

In one embodiment an increase in the production of ethylene is quantified using formula I:

$$Increase\ ETR = \frac{\int_{tx}^{ty} ETR_{experiment}}{\int_{tx}^{ty} ETR_{control}} - 1$$

wherein $\int_{tx}^{ty} ETR_{experiment}$ is the area under the curve for cells treated with the candidate substance and optionally with the compound eliciting a stress response, $\int_{tx}^{ty} ETR_{control}$ is the area under the curve for the control cells, wherein x is 1, 2 or 3 so that tx is the time point at which the candidate substance is added (t1) or the compound eliciting a stress response is added (t2) or at which the ethylene transfer rate has reached its minimum value after reaching the first maximum of the ethylene transfer rate (t3) and wherein y is 2, 3 or 4 so that ty is the time point at which the compound eliciting a stress response is added (t2) or at which the ethylene transfer rate has reached its minimum value after reaching the first maximum of the ethylene transfer rate (t3) or at which the ethylene transfer rate has reached its minimum value after reaching the second maximum of the ethylene transfer rate (t4), wherein y is greater than x.

In one embodiment an increase in the production of ethylene is quantified using formula I.1:

$$Increase\ ETR = \left( \frac{\int_{t1}^{t2} ETR_{+\ priming\ compound}}{\int_{t1}^{t2} ETR_{w/o\ additves}} - 1 \right)$$

wherein $\int_{t1}^{t2} ETR_{+\ priming\ compound}$ is the area under the curve for cells treated with the candidate substance, $\int_{t1}^{t2} ETR_{w/o\ additves}$ is the area under the curve for the untreated control cells, t1 is the time point at which the candidate substance is added and t2 is the time point at which the compound eliciting a stress response is added.

Alternatively, an increase in the production of ethylene is quantified using formula I.2:

$$Increase\ ETR = \left( \frac{\int_{t2}^{t3} ETR_{+\ priming\ compound+elicitor}}{\int_{t2}^{t3} ETR_{elicitor}} - 1 \right)$$

wherein $\int_{t2}^{t3} ETR_{+\ priming\ compound+elicitor}$ is the area under the curve for cells treated with the candidate substance and subjected to the compound eliciting a stress response, $\int_{t2}^{t3} ETR_{elicitor}$ is the area under the curve for the cells only subjected to the compound eliciting a stress response, t2 is the time point at which the compound eliciting a stress response is added and t3 is the time point at which the ethylene transfer rate has reached its minimum value after reaching the first maximum of the ethylene transfer rate and before increasing again to the second maximum of the ethylene transfer rate.

In another embodiment an increase in the production of ethylene is quantified using formula 1.3:

$$Increase\ ETR = \left( \frac{\int_{t2}^{t4} ETR_{+\ priming\ compound+elicitor}}{\int_{t2}^{t4} ETR_{elicitor}} - 1 \right)$$

wherein $\int_{t2}^{t4} ETR_{+\ priming\ compound+elicitor}$ is the area under the curve for cells treated with the candidate substance and subjected to the compound eliciting a stress response, $\int_{t2}^{t4} ETR_{elicitor}$ is the area under the curve for the cells only subjected to the compound eliciting a stress response, t2 is the time point at which the compound eliciting a stress response is added and t4 is the time point at which the ethylene transfer rate has reached its minimum value after reaching the second maximum of the ethylene transfer rate.

In still another embodiment an increase in the production of ethylene is quantified using formula 1.4:

$$Increase\ ETR = \left( \frac{\int_{t3}^{t4} ETR_{+\ priming\ compound+elicitor}}{\int_{t3}^{t4} ETR_{elicitor}} - 1 \right)$$

wherein $\int_{t3}^{t4} ETR_{+\ priming\ compound+elicitor}$ is the area under the curve for cells treated with the candidate substance and subjected to the compound eliciting a stress response, $\int_{t3}^{t4} ETR_{elicitor}$ is the area under the curve for the cells only subjected to the compound eliciting a stress response, t3 is the time point at which the ethylene transfer rate has reached its minimum value after reaching the first maximum of the ethylene transfer rate and before increasing again to the second maximum of the ethylene transfer rate and t4 is the time point at which the ethylene transfer rate has reached its minimum value after reaching the second maximum of the ethylene transfer rate.

If it is observed that the treatment with a candidate substance leads to an increase in the production of a volatile organic compound such as ethylene, but only one maximum of the ethylene transfer rate is observed, the above formula can nevertheless be used, but the period for which the area under the curve is determined is then the period between the addition of the compound eliciting a stress response and the time point at which the ethylene transfer rate has reached its minimum value after reaching the maximum.

Alternatively, an increase in the production of ethylene is quantified using formula II:

$$\Delta ETR_1 = ETR_{+priming\ compound} - ETR_{no\ additives}$$

wherein $\Delta ETR_1$ is the difference in the ethylene transfer rate, $ETR_{+priming\ compound}$ is the ethylene transfer rate for cells treated with the candidate substance after 24 hours of treatment and $ETR_{no\ additives}$ is the ethylene transfer rate for untreated control cells at the same time point.

Also alternatively, an increase in the production of ethylene is quantified using formula III:

$$\Delta ETR_2 = ETR_{+\ priming\ compound+elicitor} - ETR_{+\ elicitor}$$

wherein $\Delta ETR_2$ is the difference in the ethylene transfer rate, $ETR_{+priming\ compound+elicitor}$ is the ethylene transfer rate for cells treated with the candidate substance and subjected to the compound eliciting a stress response at the time-point when the maximum ethylene transfer rate is reached and $ETR_{+elicitor}$ is the ethylene transfer rate for cells only subjected to the treatment with the compound eliciting a stress response at the same time point.

The method may be performed for more than one volatile organic compound at the same time.

The present invention also relates to the use of said method for the characterization of signaling pathways involved in the priming of cells for a stress response by a candidate substance.

The present invention also provides a method for characterizing signaling pathways involved in priming cells for a stress response by a substance, comprising the steps of:

-    inoculating cells in a suitable culture medium and culturing them in said medium;
-    adding at least one substance to the culture medium, thereby treating the cells with said substance; and
-    measuring the production of different volatile organic compounds in the cells treated with the substance and in control cells not treated with the substance,

wherein an increase in the production of a volatile organic compound in the cells treated with the substance compared to the control cells indicates that the signaling pathway induced by the volatile organic compound is involved in the priming of cells for a stress response by said substance.

Another subject of the invention is a device comprising at least one measuring flask arranged on a vibrator table, with an inlet and an outlet for a purge gas, a valve located at the inlet and a valve located at the outlet, at least one detecting element for measuring the concentration of at least one volatile organic compound in the measuring flask and a control computer for processing the electrical signals of each detecting device.

The detecting element may be an ethylene or ethylene oxide sensor for measuring the concentration of ethylene or ethylene oxide.

The present invention also relates to the use of said device for the identification of substances which prime cells for a stress response.

## BRIEF DESCRIPTION OF THE DRAWINGS

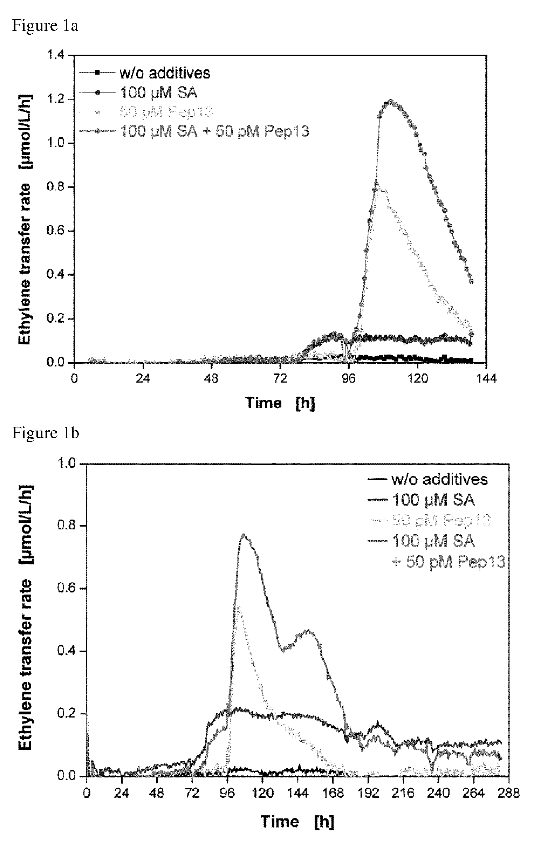

Figure 1: Ethylene transfer rate in *Petroselinum crispum* cells in cells treated with 100 $\mu$M salicylic acid which is known to be a defense priming compound and/or 50 pM Pep13 as a compound eliciting a stress response over a period of 144 hours (a) or 288 hours (b).
The graph with the black rectangles depicts the untreated culture, the graph with the dark grey diamonds depicts the culture treated with the defense priming substance, the graph with the grey triangles showing up depicts the culture treated with the compound eliciting a stress response and the graph with the light grey circles depicts the culture treated first with the defense priming substance and then additionally with the compound eliciting a stress response.

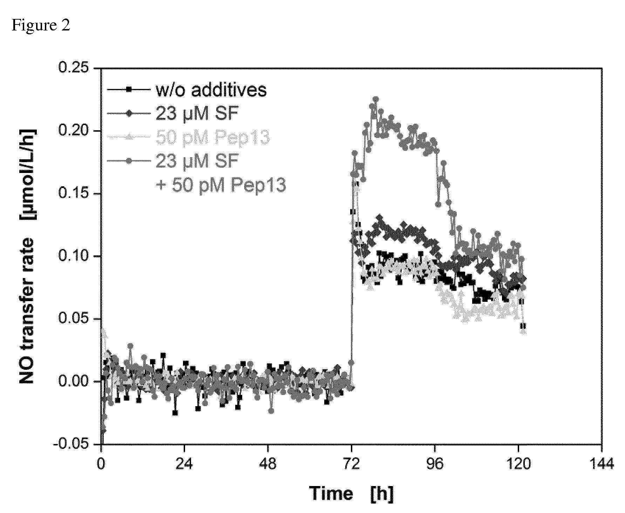

Figure 2: Nitric oxide transfer rate in cells treated with 23 $\mu$M sulforaphan as defense priming compound and/or 50 pM Pep13 as a compound eliciting a stress response over a period of 144 hours.
The graph with the black rectangles depicts the untreated culture, the graph with the dark grey diamonds depicts the culture treated with the defense priming substance, the graph with the grey triangles showing up depicts the culture treated with the compound eliciting a stress response and the graph with the light grey circles depicts the culture treated with both the defense priming substance and the compound eliciting a stress response.

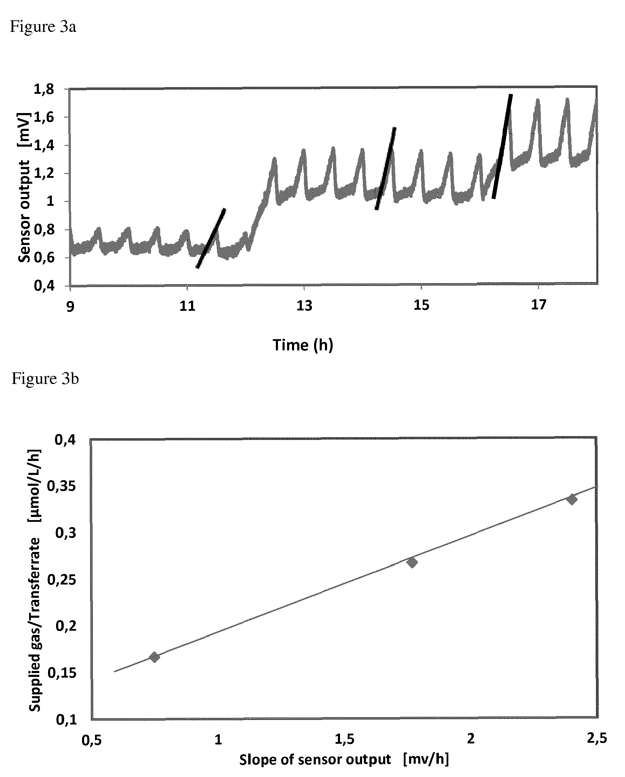

Figure 3: Method for calibrating the sensor for the volatile organic compound

a) Graph showing the signal measured by the sensor dependent on different flow rates of ethylene introduced into the shaking flask. In the left-hand part of the graph 0.17 $\mu$mol/L/h ethylene was added and a sensor output of 0.75 mV/h was measured.
In the middle part of the graph 0.27 $\mu$mol/L/h ethylene was added and a sensor output of 1.77 mV/h was measured. In the right-hand part of the graph 0.33 $\mu$mol/L/h ethylene was added and a sensor output of 2.4 mV/h was measured. The sensor output was measured in the measuring phase of the RAMOS cycle. The additional black lines indicate the slope of the sensor output for the different flow rates of ethylene.
b) Graph showing the linear correlation between the slope of the sensor output and the amount of supplied gas. This graph is used for calculating the transfer rate as explained further below.

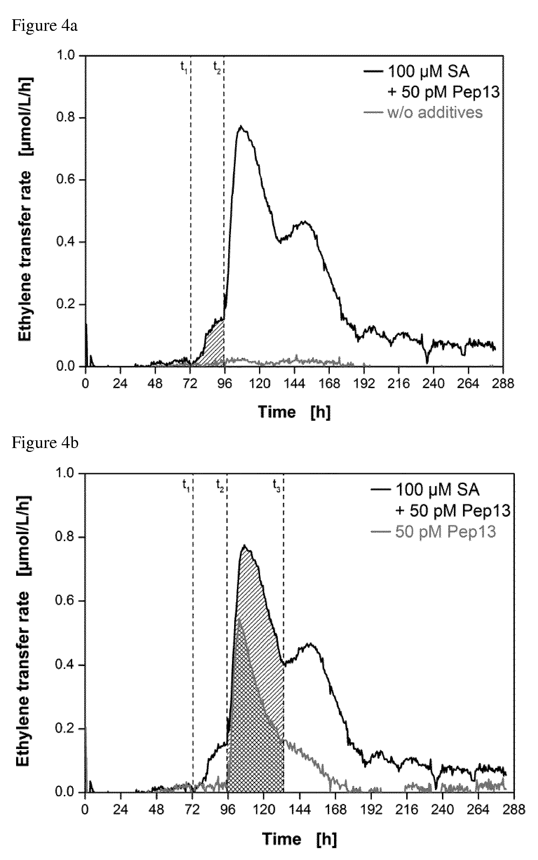

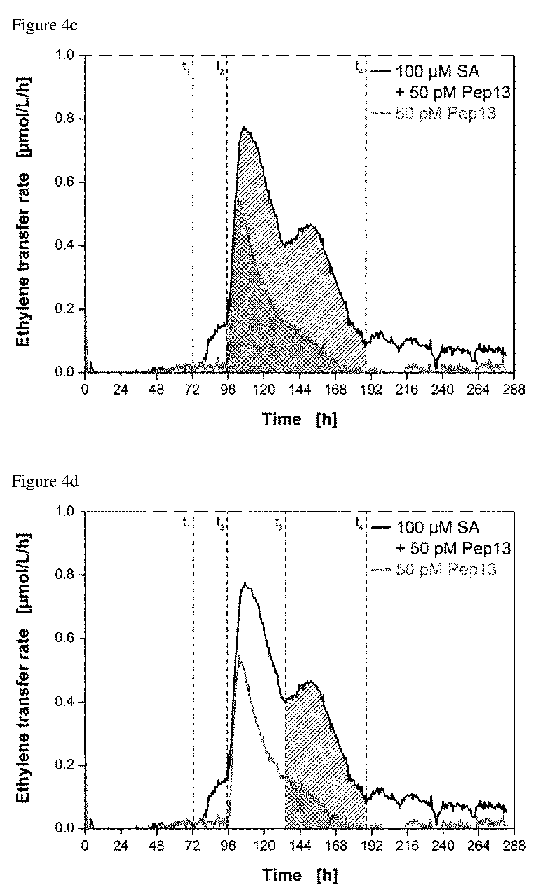

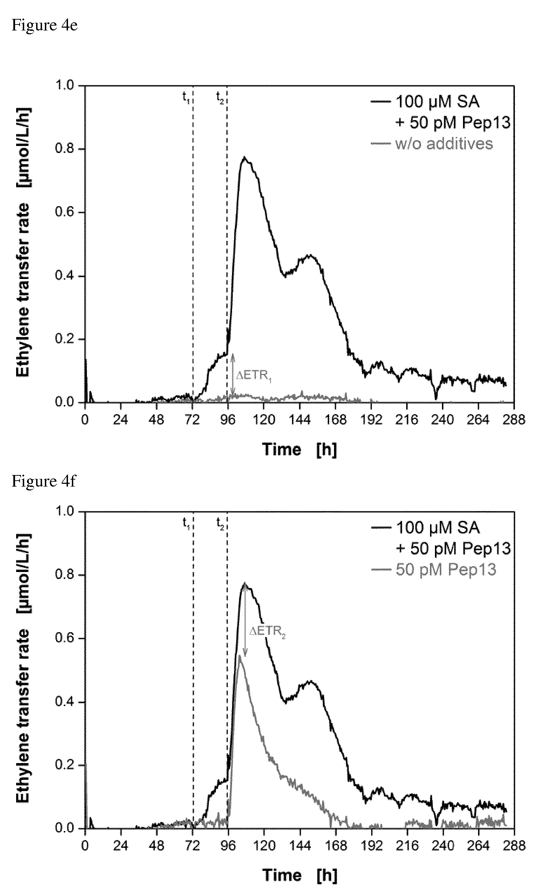

Figure 4: Different methods for calculating the increase in ethylene transfer rate after treatment with a defense priming compound.

a) Graph showing the area under the curve of the signal resulting from treatment of *Petroselinum crispum* suspension cultures with the known defense priming substance salicylic acid (hatched area) after a cultivation time of 72 hours and with the compound eliciting a stress response Pep13 after a cultivation time of 96 hours and in control cells (sketched area) in the period between adding the defense priming substance ($t_1$) and the time point at which the compound eliciting a stress response is added ($t_2$).
The black graph depicts the culture treated with the defense priming substance and the compound eliciting a stress response and the grey graph depicts the untreated culture.
b) Graph showing the area under the curve of the signal resulting from treatment of *Petroselinum crispum* suspension cultures with both the known defense priming substance salicylic acid after a cultivation time of 72 hours and with the compound eliciting a stress response Pep 13 after a cultivation time of 96 hours (hatched area) and in control cells (sketched area) in the period between adding the compound eliciting a stress response ($t_2$) and the time point at which the ethylene transfer rate has reached its minimum value after reaching the first maximum of the ethylene transfer rate and before increasing again to the second maximum of the ethylene transfer rate ($t_3$).
The black graph depicts the culture treated with the defense priming substance and the compound eliciting a stress response and the grey graph depicts the control cells only treated with the compound eliciting a stress response.
c) Graph showing the area under the curve of the signal resulting from treatment of *Petroselinum crispum* suspension cultures with both the known defense priming substance salicylic acid after a cultivation time of 72 hours and with the compound eliciting a stress response Pep 13 after a cultivation time of 96 hours (hatched area) and in control cells (sketched area) in the period between adding the compound eliciting a stress response ($t_2$) and the time point at which the ethylene transfer rate has reached its minimum value after reaching the second maximum of the ethylene transfer rate ($t_4$).
The black graph depicts the culture treated with the defense priming substance and the compound eliciting a stress response and the grey graph depicts the control cells only treated with the compound eliciting a stress response.

d) Graph showing the area under the curve of the signal resulting from treatment of *Petroselinum crispum* suspension cultures with both the known defense priming substance salicylic acid after a cultivation time of 72 hours (hatched area) and with the compound eliciting a stress response Pep13 after a cultivation time of 96 hours and in control cells (sketched area) in the period between the time point at which the ethylene transfer rate has reached its minimum value after reaching the first maximum of the ethylene transfer rate and before increasing again to the second maximum of the ethylene transfer rate ($t_3$) and the time point at which the ethylene transfer rate has reached its minimum value after reaching the second maximum of the ethylene transfer rate ($t_4$). The black graph depicts the culture treated with the defense priming substance and the compound eliciting a stress response and the grey graph depicts the control cells only treated with the compound eliciting a stress response.

e) Graph showing the increase of the ethylene transfer rate of *Petroselinum crispum* suspension cultures treated with the known defense priming substance salicylic acid and subjected to the compound eliciting a stress response Pep 13 in comparison to untreated cells ($\Delta ETR_1$).

The black graph depicts the culture treated with the defense priming substance and the compound eliciting a stress response and the grey graph depicts the untreated culture.

f) Graph showing the increase of the ethylene transfer rate of *Petroselinum crispum* suspension cultures treated with the known defense priming substance salicylic acid and subjected to the compound eliciting a stress response Pep 13 in comparison to cells only subjected to Pep13 ($\Delta ETR_2$).

The black graph depicts the culture treated with the defense priming substance and the compound eliciting a stress response and the grey graph depicts the control cells only treated with the compound eliciting a stress response.

## DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated.

The term "priming for a stress response" refers to the induction of a physiological state that enables the primed cells to respond to very low levels of a stimulus, preferably an abiotic or biotic stress, in a faster and/or stronger manner than unprimed cells. Thus, primed cells show faster and/or stronger activation of stress responses than unprimed cells when challenged by biotic or abiotic stress after priming.

Stress responses are the reaction of a cell or organism to biotic or abiotic stress and include, but are not limited to, enhanced transcription of defense genes encoding enzymes such as PAL1, PAL2, 4CL, C4H and transcription factors such as WRKY29, WRKY6 and WRKY53 and enhanced production of antimicrobial substances such as phytoalexins. Biotic stress includes the attack of a cell with a pathogen such as a virus, bacteria, fungus, insect or a nematode. Abiotic stress includes drought, heat, cold and radiation stress.

Substances which prime eukaryotic cells, in particular plant cells, for a stress response prepare the immune system of the cells for future stress. In plants which have been primed for a stress response, the stress response occurs earlier and is stronger, leading to a more effective resistance to pathogens and more effective tolerance to abiotic stress than in plants which have not been primed for a stress response.

Substances which are known to prime plant cells for a stress response include benzo(1,2,3)thiadiazole-7-carbothioic acid S-methyl ester (BTH), salicylic acid (SA), pyraclostrobin, sulforaphan and acetyl salicylic acid (aspirin).

Candidate substances are substances which should be tested for their ability to prime cells for a stress response and include natural and synthetic substances. The term "candidate substances" is also intended to include bacteria and other microorganisms which may secrete or contain substances which prime cells for a stress response. It has been shown that the colonization of Arabidopsis roots with *Pseudomonas fluorescens* bacteria primes the plant for a response to pathogens (Hase et al. (2003) Physiological and Molecular Plant Pathology 62: 219-226). The candidate substances can be tested in different concentrations to determine the minimum concentration which is required for priming the cells for a stress response.

As a positive control, another set of cells may at the same time be treated with one or more substances, from which it is known that they prime cells for a stress response, such as benzo(1,2,3)thiadiazole-7-carbothioic acid S-methyl ester (BTH), salicylic acid (SA), sulforaphan, 4-chloro salicylic acid or acetyl salicylic acid (aspirin), either alone or in combination with a compound eliciting a stress response.

As a negative control, another set of cells may at the same time be treated with one or more substances, from which it is known that they do not prime cells for a stress response, such as 3-hydroxy benzoic acid.

Those candidate substances which lead to a significant increase in the production of at least one volatile organic compound, preferably of ethylene, in the treated cells in comparison to the untreated control cells or the negative control cells are substances which prime the cells for a stress response.

After identifying substances which are capable of priming a stress response by the method of the present invention, it can be confirmed that these substances prime the cells for a stress response by treatment of plants with this substance and then exposing the plants to a pathogen or abiotic stress and measuring the stress response. It is confirmed that a substance primes cells for a stress response, if after treatment with the substance and exposing the plants to a pathogen or abiotic stress the stress response in the plant treated with the substance occurs earlier and/or is stronger than in a plant not treated with the substance.

If the cells used in the method of the present invention are parsley (*Petroselinum crispum*) cells, the priming effect of the candidate substance can also be confirmed by detection of fluorescent substances which are secreted from the parsley cells upon treatment with a compound eliciting a stress response as described in Siegrist et al. (1998) Physiol. Mol. Plant Pathol. 53(4): 223-238. The fluorescence is measured 6 to 48 hours, preferably 12 to 40 hours, more preferably 16 to 36 hours, even more preferably 20 to 32 hours and most preferably 24 hours after addition of the compound eliciting a stress response.

The cells which are used in the method of the present invention may be plant or animal cells, such as mammalian cells. Preferably, plant cells are used. The plant cells may be derived from a monocotyledonous or dicotyledonous plant.

Examples of monocotyledonous plants are plants belonging to the genera Avena (oat), Triticum (wheat), Secale (rye), Hordeum (barley), Oryza (rice), Panicum, Pennisetum, Setaria, Sorghum (millet), Zea (maize), and the like.

Dicotyledonous plants comprise, *inter alia,* Arabidopsis, cotton, legumes, like leguminous plants and in particular alfalfa, soybean, rape, canola, tomato, sugar beet, potato, ornamental plants, and trees. Further dicotyledonous plants can comprise fruit (in particular apples, pears, cherries, grapes, citrus, pineapple, and bananas), pumpkin, cucumber, wine, oil palms, tea shrubs, cacao trees, and coffee shrubs, tobacco, sisal, as well as, with medicinal plants, rauwolfia and digitalis.

Particularly preferred for use in the method of the present invention are cells from agronomically important plant species, since the substances identified by the method of the present invention can then directly be used for the treatment of plants of this species. Also particularly preferred are cells from plant species which show a uniform growth profile such as *Petroselinum crispum.*

Most preferably, the cells are from *Petroselinum crispum, Triticum aestivum, Solanum turberosum, Oryza sativa, Solanum turberosum, Glycine max* or *Zea mays.*

Suitable mammalian cells include, but are not limited to, human, mouse, rat, hamster, bovine and porcine cells. Preferably the cells are rodent or human cells. Examples of suitable mammalian cells include HeLa, NIH3T3, CHO and 293 cells.

The term "inoculating" means that the cells which are used in the method of the present invention are brought into contact with the culture medium.

The term "culturing" is intended to mean that the cells are held under conditions such as medium, temperature and pH which allow the maintenance and proliferation of the cells. For plant cells such as *Petroselinum crispum* cells, the medium may be modified Gamborg B5 medium and the temperature may be 25°C. In the method of the present invention the cells are preferably grown in suspension culture, i.e. without being attached to a substrate. More preferably, the cells are grown in shaking flasks, such as a shake flask with a nominal volume of 100 to 500 mL with a filling volume of 1/20 to 1/4 of the nominal volume, a shaking diameter of 12.5 to 100 mm and a shaking frequency of 150 to 400 rpm. Most preferably, the cells are grown in a shake flask with a nominal volume of 250 mL, a filling volume of 50 mL, a shaking diameter of 5 cm and a shaking frequency of 180 rpm.

"Culture medium" is used for the maintenance of cells in culture *in vitro.* For some cell types, the medium may also be sufficient to support the proliferation of the cells in culture. A culture medium typically provides nutrients such as energy sources, amino acids and inorganic ions. Additionally, it may contain a dye like phenol red, sodium pyruvate, several vitamins, free fatty acids, antibiotics, anti-oxidants, minerals and trace elements.

For culturing plant cells a number of media is available, including CHU ($N_6$) medium, Gamborg B5 medium, Murashige & Skoog Medium (MS), Murashige & Skoog Modified Medium and Schenk & Hildebrandt Medium (SH). These media are well-known to a person skilled in the art and may be purchased from companies such as Sigma-Aldrich (Deisenhofen, Germany) and PhytoTechnology Laboratories (Shawnee Mission, USA).

For culturing mammalian cells any standard medium such as Iscove's Modified Dulbecco's Media (IMDM), alpha-MEM, Dulbecco's Modified Eagle Media (DMEM), RPMI Media and McCoy's Medium may be used. These media are well-known to a person skilled in the art and may be purchased from companies such as Cambrex (East Rutherford, USA), Invitrogen (San Diego, USA) and Sigma-Aldrich (Deisenhofen,Germany).

The term "volatile organic compound" (hereinafter abbreviated as VOC) refers to substances which are in the gaseous form under the culture conditions used. Preferably, the volatile organic compounds are evaporated from the cells in response to treatment with pathogens and are involved in pathogen defense. In particular, this term includes ethylene and ethylene oxide, nitrogen oxide, hydrogen peroxide and jasmonate.

The production of the volatile organic compound can be determined by any method known in the art. Preferably, the production of the volatile organic compound is determined by a modified respiration activity monitoring system (modRAMOS). This system was originally developed for monitoring the respiration activity of cells by measuring the oxygen and/or carbon dioxide transfer rate. It is described in German patent application DE 44 15 444 and in European patent application EP 0 905 229 A2 as well as in Anderlei and Buechs (2001) Biochemical Engineering Journal 7: 157-162 and Anderlei et al. (2004) Biochemical Engineering Journal 17: 187-194. A suitable RAMOS device is commercially available from HiTec Zang GmbH (Herzogenrath, Germany) and from Kuhner AG (Birsfelden, Switzerland).

In general, the RAMOS system comprises at least one measuring flask arranged on a shaking tray, with an inlet and an outlet for a purge gas, a valve located at the inlet and a valve located at the outlet, at least one detecting element for measuring the concentration of at least one gaseous substance in the measuring flask and a control computer for processing the electrical signals of each detecting device. Within the present invention the term "detecting element" is to be understood to be equivalent to the term "sensor".

For the method of the present invention the RAMOS system is modified to allow for the measurement of the at least one volatile organic compound, e.g. by installing a sensor for said volatile organic compound. Apart from this modification the RAMOS system is the same as described in German patent DE 44 15 444 and in European patent application EP 0 905 229 A2. It is possible to measure the production of several volatile organic compounds in the same experiment by equipping the same RAMOS system with more than one sensor, wherein each sensor is suitable for detecting a particular volatile organic compound. Care should be taken that the sensor used does not show cross-sensitivity for other volatile organic compounds or at least that this cross-sensitivity is as low as possible. Of course, it is also possible to combine the measurement of the production of the at least one volatile organic compound with the measurement of the respiration activity of the cells as described in international patent application PCT/EP2015/069593 by combining at least one sensor for a volatile organic compound with a sensor for oxygen and/or carbon dioxide.

Suitable sensors for measuring the concentration of ethylene in a sample include C2H4-M-10 available from Membrapor, 8304 Wallisellen, Switzerland, EC4-10-ETO available from SGX Sensortech, Chelmsford, United Kingdom and ETO-A1 available from Alphasense, Great Notley, United Kingdom. The production of nitric oxide can be measured with NO-A1 available from Alphasense, Great Notley, United Kingdom.

In the RAMOS system, a measuring cycle is continuously repeated during cultivation of the cells which are treated with the candidate substance. This measuring cycle comprises a measuring phase and a rinsing phase. During the rinsing phase, air is flushed through the measuring flask at a specific flow rate. At the beginning of the measuring phase, inlet and outlet valves of the measuring flask are closed. If the cells produce at least one volatile organic compound such as ethylene, the partial pressure of this substance in the gas headspace of the measuring flask will increase. This partial pressure is monitored with a suitable sensor as described above.

Before measuring the production of the at least one volatile organic compound the sensor for the at least one volatile organic compound has to be calibrated to enable a quantitative analysis of the production of the at least one volatile organic compound.

For the calibration of the sensor for the at least one volatile substances several methods are available. In a preferred embodiment, a defined flow rate of the volatile organic compound, preferably of ethylene, is introduced into the shaking flask, when the inlet and outlet valves for the purge gas are closed. Then the partial pressure of the volatile organic compound, preferably of ethylene, is measured using the detecting element/sensor. This measurement is repeated with different defined flow rates of the volatile organic compound, preferably of ethylene. The sensor signals obtained over time are shown in Figure 3a. This graph allows to calculate the slope of the signal curve obtained with each defined flow rate of the volatile organic compound. When the slope of the signal curve is then plotted against the known flow rate of the volatile organic compound introduced into the flask, a linear correlation between the slope of the signal curve and the flow rate of the volatile organic compound can be observed (see Figure 3b).

The transfer rate of the volatile organic compound, preferably of ethylene, can then be determined using the following formula:

$$ETR = c \times \frac{dU_{C2H4}}{dt} + d$$

wherein ETR is the ethylene transfer rate, c is the slope of the calibration line, $\frac{dU_{C2H4}}{dt}$ is the change in sensor voltage dependent on the ethylene flow rate over time and d is the intercept of the calibration line.

In the method of the present invention the production of the at least one volatile organic compound of the cells treated with the candidate substance is compared with the production of the at least one volatile organic compound of control cells not treated with the candidate substance. These control cells can be (a) cells in the culture medium which are not treated with any substance, if the treated cells are only treated with the candidate substance, but not with a compound eliciting a stress response, or (b) cells subjected to a compound eliciting a stress response, if the cells treated with the candidate substance are also subjected to said compound eliciting a stress response.

A candidate substance primes cells for a stress response, if the treatment of the cells with the candidate substance leads to an increased production of at least one volatile organic compound, in particular an increased ethylene production, compared to the production of this substance, in particular ethylene, in cells not treated with the candidate substance.

The candidate substance is added to the culture medium after a cultivation time of 24 to 120 hours, preferably of 36 to 100 hours, more preferably of 48 to 90 hours, even more preferably of 60 to 80 hours and most preferably of 72 hours.

In one embodiment of the present invention the cells may be subjected to a compound eliciting a stress response. The elicitor of a stress response is any substance, organism or environmental condition which elicits a stress response within a cell, in particular a plant cell. Abiotic elicitors for plant cells include drought, heat, cold and radiation stress. Biotic elicitors for plant cells include pathogens such as fungi, bacteria, nematodes, insects and viruses.

The compound eliciting a stress response may also be a pathogen-associated molecular pattern (PAMP) which is a small molecular motif of pathogens, but not of the recipient organism. It is recognized by toll-like receptors and other pattern recognition receptors in animal and plant cells and then induces a defense response within the cell. Suitable PAMPs are known to the expert and include Pep-13 (Nürnberger et al. (1994) Cell 78: 449-460), NPP1 (Fellbrich et al. (2002) Plant J. 32(3): 375-390), flg22 (Felix et al. (1999) Plant J. 18(3): 265-276), EF-Tu (Zipfel et al. (2006) Cell 125: 749-760), CBEL (Villalba-Mateos et al. (1997) Mol. Plant Microbe Interact. 10: 1045-1053), AsES (Chalfoun et al. (2013) J. Biol. Chem. 288(20): 14098-14113) and TLKGE (Rotblat et al. (2002) Plant J. 32(6): 1049-1055).

The cells are subjected to the compound eliciting a stress response 6 to 48 hours, preferably 12 to 40 hours, more preferably 16 to 36 hours, even more preferably 20 to 32 hours and most preferably 24 hours after addition of the candidate substance.

Any difference in the production of the volatile organic compound, preferably of ethylene, between the cells treated with the candidate substance and the untreated control cells can be visualized by plotting the VOC transfer rate, preferably the ethylene transfer rate, measured in mmol/L/h (on the y axis) against the culture time (on the x axis) for the cells treated with the candidate substance and the control cells not treated with the candidate substance. Figures 1 and 2 show that the treatment of cells with substances from which it is known that they prime cells for a stress response increases the ethylene transfer rate and the nitrogen oxide transfer rate, respectively, in a characteristic manner.

The difference in production of the at least one volatile organic compound, preferably of ethylene, between the cells treated with the candidate substance and the control cells not treated with the candidate substance can be quantified by several calculations, used either alone or in combination. Generally, it is sufficient, if a candidate substance leads to a significant increase of the VOC transfer rate, in particular the ethylene transfer rate, in any of these calculations, but a combination of two or more calculation methods may provide more detailed information.

A first possibility to quantify any increase in the production of a volatile organic compound, in particular of ethylene, is to determine the increase in production as the difference of the area of the VOC transfer rate of the cells treated with the candidate substance and the VOC transfer rate of the untreated control cells in the period after adding the candidate substance until the cells are subjected to a compound eliciting a stress response. This calculation is illustrated in Figure 4a, wherein the relative area between the hatched area and the checked area is determined.

The increase of ethylene production may thus be determined using the following formula I:

$$Increase\ ETR = \frac{\int_{tx}^{ty} ETR_{experiment}}{\int_{tx}^{ty} ETR_{control}} - 1$$

wherein $\int_{tx}^{ty} ETR_{experiment}$ is the area under the curve for cells treated with the candidate substance and optionally with the compound eliciting a stress response, $\int_{tx}^{ty} ETR_{control}$ is the area under the curve for the control cells, wherein x is 1, 2 or 3 so that tx is the time point at which the candidate substance is added (t1) or the compound eliciting a stress response is added (t2) or at which the ethylene transfer rate has reached its minimum value after reaching the first maximum of the ethylene transfer rate (t3) and wherein y is 2, 3 or 4 so that ty is the time point at which the compound eliciting a stress response is added (t2) or at which the ethylene transfer rate has reached its minimum value after reaching the first maximum of the ethylene transfer rate (t3) or at which the ethylene transfer rate has reached its minimum value after reaching the second maximum of the ethylene transfer rate (t4), wherein y is greater than x.

The control cells are the untreated cells, if the cells of the experiment are only treated with the candidate substance. Alternatively, the control cells are cells treated only with the compound eliciting a stress response, if the cells of the experiment are treated with both the candidate substance and the compound eliciting a stress response.

The time points t1, t2, t3 and t4 are those indicated in Figure 4d.

In one embodiment an increase in the production of ethylene is quantified using formula I.1:

$$Increase\ ETR = \left( \frac{\int_{t1}^{t2} ETR_{+\ priming\ compound}}{\int_{t1}^{t2} ETR_{w/o\ additves}} - 1 \right)$$

wherein $\int_{t1}^{t2} ETR_{+\ priming\ compound}$ is the area under the curve for cells treated with the candidate substance, $\int_{t1}^{t2} ETR_{w/o\ additves}$ is the area under the curve for the untreated control cells, t1 is the time point at which the candidate substance is added and t2 is the time point at which the compound eliciting a stress response is added.

Alternatively, the increase in the ethylene transfer rate may be calculated using Formula IV:

$$Increase\ in\ ETR = \frac{\int_{t1}^{t2} ETR_{+\ priming\ compound} - \int_{t1}^{t2} ETR_{w/oadditves}}{(t2 - t1)}$$

wherein $\int_{t1}^{t2} ETR_{+\ priming\ compound}$ is the area under the curve for cells treated with the candidate substance, $\int_{t1}^{t2} ETR_{w/o\ additves}$ is the area under the curve for the untreated control cells, t1 is the time point at which the candidate substance is added and t2 is the time point at which the compound eliciting a stress response is added.

A candidate substance is considered to prime cells for a stress response, if the increase in ETR calculated using the above formula IV is at least 0.02 µmol/L/h or 0.03 µmol/L/h, preferably at least 0.04 µmol/L/h or 0.05 µmol/L/h, more preferably at least 0.06 µmol/L/h or 0.07 µmol/L/h, even more preferably at least 0.08 µmol/L/h or 0.09 µmol/L/h and most preferably at least 0.1 µmol/L/h.

A second possibility to quantify any increase in the production of a volatile organic compound, in particular of ethylene, is to determine the increase in production as the relative area of the VOC transfer rate of the cells treated with the candidate substance and the compound eliciting a stress response and the VOC transfer rate of the control cells only treated with the compound eliciting a stress response in the period after adding the compound eliciting a stress response until the VOC transfer rate has reached its minimum after the first maximum of the VOC transfer rate and before increasing to the second maximum of the VOC transfer rate. This calculation is illustrated in Figure 4b, wherein the relative area between the hatched area and the checked area is determined.

The increase of ethylene production may thus be determined using the following formula 1.2:

$$Increase\ ETR = \left( \frac{\int_{t2}^{t3} ETR_{+\ priming\ compound+elicitor}}{\int_{t2}^{t3} ETR_{+\ elicitor}} - 1 \right)$$

wherein $\int_{t2}^{t3} ETR_{+\ priming\ compound+elicitor}$ is the area under the curve for cells treated with the candidate substance and subjected to the compound eliciting a stress response, $\int_{t2}^{t3} ETR_{+elicitor}$ is the area under the curve for the cells subjected only to the compound eliciting a stress response, t2 is the time point at which the compound eliciting a stress response is added and t3 is the time point at which the ethylene transfer rate has reached its minimum value after reaching the first maximum of the ethylene transfer rate and before increasing again to the second maximum of the ethylene transfer rate.

A candidate substance is considered to prime cells for a stress response, if the increase in ETR calculated using the above formula I.2 is at least 10% or 15%, preferably at least 20% or 25%, more preferably at least 30% or 35%, even more preferably at least 40% or 45% and most preferably at least 50%.

A third possibility to quantify any increase in the production of a volatile organic compound, in particular of ethylene, is to determine the increase in production as the relative area of the VOC transfer rate of the cells treated with the candidate substance and the compound eliciting a stress response and the VOC transfer rate of the control cells only treated with the compound eliciting a stress response in the period after adding the compound eliciting a stress response until the VOC transfer rate has reached its minimum after the second maximum of the VOC transfer rate. This calculation is illustrated in Figure 4c, wherein the relative area between the hatched area and the checked area is determined.

The increase of ethylene production may thus be determined using the following formula 1.3:

$$Increase\ ETR = \left( \frac{\int_{t2}^{t4} ETR_{+\ priming\ compound+elicitor}}{\int_{t2}^{t4} ETR_{+\ elicitor}} - 1 \right)$$

wherein $\int_{t2}^{t4} ETR_{+\ priming\ compound+elicitor}$ is the area under the curve for cells treated with the candidate substance and subjected to the compound eliciting a stress response, $\int_{t2}^{t4} ETR_{+\ elicitor}$ is the area under the curve for the cells only subjected to the compound eliciting a stress response, t2 is the time point at which the compound eliciting a stress response is added and t4 is the time point at which the ethylene transfer rate has reached its minimum value after reaching the second maximum of the ethylene transfer rate.

A candidate substance is considered to prime cells for a stress response, if the increase in ethylene production calculated using the above formula 1.3 is at least 10% or 20%, preferably at least 30% or 40%, more preferably at least 45% or 50%, even more preferably at least 55% or 60% and most preferably at least 65% or 70%.

A fourth possibility to quantify any increase in the production of a volatile organic compound, in particular of ethylene, is to determine the increase in production as the relative area of the VOC transfer rate of the cells treated with the candidate substance and the compound eliciting a stress response and the VOC transfer rate of the control cells only treated with the compound eliciting a stress response in the period between the time point at which the VOC transfer rate has reached its minimum value after reaching the first maximum of the VOC transfer rate and before increasing again to the second maximum of the VOC transfer rate and the time point at which the VOC transfer rate has reached its minimum after the second maximum of the VOC transfer rate. This calculation is illustrated in Figure 4d, wherein the relative area between the hatched area and the checked area is determined.

The increase of ethylene production may thus be determined using the following formula 1.4:

$$Increase\ ETR = \left( \frac{\int_{t3}^{t4} ETR_{+\ priming\ compound+elicitor}}{\int_{t3}^{t4} ETR_{+\ elicitor}} - 1 \right)$$

wherein $\int_{t3}^{t4} ETR_{+\ priming\ compound+elicitor}$ is the area under the curve for cells treated with the candidate substance and subjected to the compound eliciting a stress response, $\int_{t3}^{t4} ETR_{+elicitor}$ is the area under the curve for the cells only subjected to the compound eliciting a stress response, t3 is the time point at which the ethylene transfer rate has reached its minimum value after reaching the first maximum of the ethylene transfer rate and before increasing again to the second maximum of the ethylene transfer rate and t4 is the time point at which the ethylene transfer rate has reached its minimum value after reaching the second maximum of the ethylene transfer rate.

A candidate substance is considered to prime cells for a stress response, if the increase in ethylene production calculated using the above formula I.4 is at least 20% or 30%, preferably at least 40% or 50%, more preferably at least 60% or 70%, even more preferably at least 80% or 90% and most preferably at least 100%.

A fifth possibility to quantify any increase in the production of a volatile organic compound, in particular of ethylene, is to determine the difference in the VOC transfer rate, preferably the ethylene transfer rate, in cells treated with the candidate substance and the untreated control cells at a specific time-point, e.g. after 24 hours of treatment with the candidate substance. This calculation is illustrated in Figure 4e, wherein $\Delta ETR_1$ is the difference in the ethylene transfer rate.

The increase of ethylene production may thus be determined using the following formula II:

$$\Delta ETR_1 = ETR_{+priming\ compound} - ETR_{w/o\ additives}$$

wherein $\Delta ETR_1$ is the difference in the ethylene transfer rate, $ETR_{+priming\ compound}$ is the ethylene transfer rate for cells treated with the candidate substance before being subjected to an elicitor of a stress response after a certain period of treatment with the candidate substance, preferably after 24 hours, and $ETR_{no\ additives}$ is the ethylene transfer rate for untreated control cells at the same time point.

A candidate substance is considered to prime cells for a stress response, if the difference in the ethylene transfer rate is at least 0.02 $\mu$mol/L/h, preferably at least 0.04 $\mu$mol/L/h, more preferably at least 0.06 $\mu$mol/L/h and most preferably at least 0.08 $\mu$mol/L/h.

A sixth possibility to quantify any increase in the production of a volatile organic compound, in particular of ethylene, is to determine the difference in the ethylene transfer rate in cells treated with the candidate substance and the compound eliciting a stress response and the ethylene transfer rate in cells subjected only to the compound eliciting a stress response at a specific time-point, e.g. at the first maximum of the ethylene transfer rate. This calculation is illustrated in Figure 4f, wherein $\Delta ETR_2$ is the difference in the ethylene transfer rate.

The increase of ethylene production may thus be determined using the following formula III:

$$\Delta ETR_2 = ETR_{+\ priming\ compound+elicitor} - ETR_{+\ elicitor}$$

wherein $\Delta ETR_2$ is the difference in the ethylene transfer rate, $ETR_{+priming\ compound+elicitor}$ is the ethylene transfer rate for cells treated with the candidate substance and subjected to the compound eliciting a stress response at the time-point when the maximum ethylene transfer rate is reached and $ETR_{+elicitor}$ is the ethylene transfer rate for cells only subjected to the treatment with the compound eliciting a stress response at the same time point.

A candidate substance is considered to prime cells for a stress response, if the difference in the ethylene transfer rate is at least 0.03 $\mu$mol/L/h, preferably at least 0.06 $\mu$mol/L/h, more preferably at least 0.08 $\mu$mol/L/h and most preferably at least 1.0 $\mu$mol/L/h.

In the plotting of the VOC transfer rate, preferably the ethylene transfer rate, versus time of cells cultured under different conditions in one graph it may happen that due to practical reasons the curves from inoculating the cell culture until the addition of the candidate substance do not have the same values. In this case, it may be necessary to align the curves to have the same starting conditions for all conditions investigated. This can be done by adjusting the position of one or more curves on the y axis so that ultimately the curves are superimposed. To this end, the sum of all differences between all reading points in the period from inoculating the culture until the addition of the candidate substance is calculated. Then different values are added to the absolute y values of the curve to be aligned using a known iterative method such as Excel Solver, until the sum of the differences between all reading points is minimal.

The method of the present invention can also be used to characterize the signaling pathways involved in priming cells for a stress response by a substance. In this case, the cells may be treated either with a substance of which it is known to prime cells for a stress response or with a candidate substance. Preferably, the production of more than one volatile organic compound by the cells is measured. This can be accomplished either by treating different cell samples with the substance and measuring the production of one volatile organic compound per sample or by treating one cell sample with the substance and measuring the production of more than one volatile organic compound in said sample by using sensors for the different volatile organic compounds in parallel. In this case, an increase in the production of a volatile organic compound indicates that the signaling pathway induced by the volatile organic compound is involved in priming the cells for a stress response.

The identification of substances which prime for a stress response by measuring the production of volatile organic compounds is described in the following examples:

**EXAMPLES**

1. <u>Increase of the ethylene transfer rate upon treatment with a substance which primes the cells for a stress response</u>

12.5 ml of a suspension of *Petroselinum crispum* cells were inoculated in 250 ml shaking flasks containing 37.5 ml of modified Gamborg B5 medium and cultured at a temperature of 25°C at a shaking frequency of 180U/min and a shaking diameter of 5cm in a RAMOS device (HiTec Zang, Herzogenrath) equipped with an ethylene sensor (C2H4-M-10 of Membrapor, 8304 Wallisellen, Switzerland) or a nitric oxide sensor (NO-A1 of Alphasense). During the culture the ethylene or nitric oxide partial pressure of the gas phase in the head space of the flask was measured and the ethylene transfer rate or nitric oxide transfer rate was calculated therefrom.

72 hours after inoculation of the cells the known defense priming substance salicylic acid was added at a concentration of 100 $\mu$M. Alternatively, the cells were treated with the known defense priming substance sulforaphan at a concentration of 23 $\mu$M. After another 24 hours (i.e. 96 hours after inoculation), 50nM of Pep13 which elicits a stress response of *Petroselinum crispum* cells was added to the culture. The ethylene transfer rate was determined for cells treated with salicylic acid and Pep13 and is shown in Figure 1. The nitric oxide transfer rate was determined for cells treated with sulforaphan and Pep13 and is shown in Figure 2.

These data show that the treatment of cells with a substance which primes the cells for a stress response leads to an increase in the production of the volatile organic compounds ethylene and nitric oxide in comparison to untreated cells and to cells only treated with a compound eliciting a stress response.

Some embodiments of the present invention relate to:

1. Method for identifying substances which prime cells for a stress response comprising the steps of:

- inoculating cells in a suitable culture medium and culturing them in said medium;
- adding one or more candidate substances to the culture medium thereby treating the cells with said candidate substance; and
- measuring the production of at least one volatile organic compound in the cells treated with the candidate substance and in control cells not treated with the candidate substance,

wherein an increase in the production of the at least one volatile organic compound in the cells treated with the candidate substance compared to the control cells indicates that the candidate substance primes the cells for a stress response.

2. Method according to item 1, wherein the at least one volatile organic compound is selected from the group consisting of ethylene, nitric oxide and hydrogen peroxide.

3. Method according to item 1 or 2, wherein the production of the at least one volatile substance is measured in a shaken measuring flask under sterile conditions, wherein during a purge phase air passes through the measuring flask and in a measuring phase the air stream is interrupted and the concentration of the at least one volatile organic compound is measured with a detecting element for said at least one volatile organic compound.

4. Method according to item 3, further comprising calibrating the detecting element.

5. Method according to item 4, wherein the detecting element is calibrated by passing air with a defined concentration of the at least one volatile organic compound through the measuring flask during the purge phase and measuring the concentration of the at least one volatile organic compound with the detecting element.

6. Method for identifying substances which prime plant cells for a stress response comprising the steps of:

- inoculating plant cells in a suitable culture medium and culturing them in said medium;
- adding one or more candidate substances to the culture medium thereby treating the plant cells with said candidate substance; and
- measuring the production of ethylene in the cells treated with the candidate substance and in control cells not treated with the candidate substance,

wherein an increase in the production of ethylene in the cells treated with the candidate substance compared to the control cells indicates that the candidate substance primes the plant cells for a stress response.

7. Method according to item 6, wherein the production of ethylene is measured in a shaken measuring flask under sterile conditions, wherein during a purge phase air passes through the measuring flask and in a measuring phase the air stream is interrupted and the concentration of ethylene is measured with a detecting element for ethylene.

8. Method according to item 7, further comprising calibrating the detecting element.

9. Method according to item 8, wherein the detecting element is calibrated by passing a defined flow rate of ethylene through the measuring flask during the purge phase and measuring the concentration of ethylene with the detecting element.

10. Method according to any one of the preceding items, wherein the candidate substance is added 24 to 120 hours after inoculation.

11. Method according to any one of the preceding items, further comprising, after the addition of the candidate substance, subjecting the cells to a compound eliciting a stress response.

12. Method according to item 11, wherein the cells are subjected to the compound eliciting a stress response 6 to 48 hours after the addition of the candidate substance.

13. Method according to any one of items 2 to 12, wherein an increase in the production of ethylene is quantified using formula I.1:

$$Increase\ ETR = \left( \frac{\int_{t1}^{t2} ETR_{+\ priming\ compound}}{\int_{t1}^{t2} ETR_{w/o\ additves}} - 1 \right)$$

wherein $\int_{t1}^{t2} ETR_{+\ priming\ compound}$ is the area under the curve for cells treated with the candidate substance,

$\int_{t1}^{t2} ETR_{w/o\ additves}$ is the area under the curve for the untreated control cells, t1 is the time point at which the candidate substance is added and t2 is the time point at which the compound eliciting a stress response is added.

14. Method according to any one of items 2 to 12, wherein an increase in the production of ethylene is quantified using formula I.2:

$$Increase\ ETR = \left( \frac{\int_{t2}^{t3} ETR_{+\ priming\ compound+elicitor}}{\int_{t2}^{t3} ETR_{elicitor}} - 1 \right)$$

wherein $\int_{t2}^{t3} ETR_{+\ priming\ compound+elicitor}$ is the area under the curve for cells treated with the candidate substance and subjected to the compound eliciting a stress response, $\int_{t2}^{t3} ETR_{elicitor}$ is the area under the curve for the cells only subjected to the compound eliciting a stress response, t2 is the time point at which the compound eliciting a stress response is added and t3 is the time point at which the ethylene transfer rate has reached its minimum value after reaching the first maximum of the ethylene transfer rate and before increasing again to the second maximum of the ethylene transfer rate.

15. Method according to any one of items 2 to 12, wherein an increase in the production of ethylene is quantified using formula I.3:

$$Increase\ ETR = \left( \frac{\int_{t2}^{t4} ETR_{+\ priming\ compound+elicitor}}{\int_{t2}^{t4} ETR_{elicitor}} - 1 \right)$$

wherein $\int_{t2}^{t4} ETR_{+\ priming\ compound+elicitor}$ is the area under the curve for cells treated with the candidate substance and subjected to the compound eliciting a stress response, $\int_{t2}^{t4} ETR_{elicitor}$ is the area under the curve for the cells only subjected to the compound eliciting a stress response, t2 is the time point at which the compound eliciting a stress response is added and t4 is the time point at which the ethylene transfer rate has reached its minimum value after reaching the second maximum of the ethylene transfer rate.

16. Method according to any one of items 2 to 12, wherein an increase in the production of ethylene is quantified using formula I.4:

$$Increase\ ETR = \left( \frac{\int_{t3}^{t4} ETR_{+\ priming\ compound+elicitor}}{\int_{t3}^{t4} ETR_{elicitor}} - 1 \right)$$

wherein $\int_{t3}^{t4} ETR_{+\ priming\ compound+elicitor}$ is the area under the curve for cells treated with the candidate substance and subjected to the compound eliciting a stress response, $\int_{t3}^{t4} ETR_{elicitor}$ is the area under the curve for the cells only subjected to the compound eliciting a stress response, t3 is the time point at which the ethylene transfer rate has reached its minimum value after reaching the first maximum of the ethylene transfer rate and before increasing again to the second maximum of the ethylene transfer rate and t4 is the time point at which the ethylene transfer rate has reached its minimum value after reaching the second maximum of the ethylene transfer rate.

17. Method according to any one of items 2 to 12, wherein an increase in the production of ethylene is quantified using formula II:

$$\Delta ETR_1 = ETR_{+priming\ compound} - ETR_{no\ additives}$$

wherein $\Delta ETR_1$ is the difference in the ethylene transfer rate, $ETR_{+priming\ compound}$ is the ethylene transfer rate for cells treated with the candidate substance after 24 hours of treatment and $ETR_{no\ additives}$ is the ethylene transfer rate for untreated control cells at the same time point.

18. Method according to any one of items 2 to 12, wherein an increase in the production of ethylene is quantified using formula III:

$$\Delta ETR_2 = ETR_{+\ priming\ compound+elicitor} - ETR_{+\ elicitor}$$

wherein $\Delta ETR_2$ is the difference in the ethylene transfer rate, $ETR_{+priming\ compound+elicitor}$ is the ethylene transfer rate for cells treated with the candidate substance and subjected to the compound eliciting a stress response at the time-point when the maximum ethylene transfer rate is reached and $ETR_{+elicitor}$ is the ethylene transfer rate for cells only subjected to the treatment with the compound eliciting a stress response at the same time point.

19. Method according to any one of items 1 to 18, wherein the method is performed for more than one volatile organic compound in parallel.

20. Use of the method according to item 19 for the characterisation of signaling pathways involved in the priming of cells for a stress response by a candidate substance.

21. Method for characterizing signaling pathways involved in priming cells for a stress response by a substance, comprising the steps of:

- inoculating cells in a suitable culture medium and culturing them in said medium;
- adding at least one substance to the culture medium, thereby treating the cells with said substance; and
- measuring the production of different volatile organic compounds in the cells treated with the substance and in control cells not treated with the substance,

wherein an increase in the production of a volatile organic compound in the cells treated with the substance compared to the control cells indicates that the signaling pathway induced by the volatile organic compound is involved in the priming of cells for a stress response by said substance.

22. Device comprising at least one measuring flask arranged on a vibrator table, with an inlet and an outlet for a purge gas, a valve located at the inlet and a valve located at the outlet, at least one detecting element for measuring the concentration of at least one volatile organic compound in the measuring flask and a control computer for processing the electrical signals of each detecting device.

23. Device according to item 22, wherein the detecting element is an ethylene or ethylene oxide sensor for measuring the concentration of ethylene or ethylene oxide.

24. Use of the device according to item 22 or 23 for the identification of substances which prime cells for a stress response.

## Claims

1. Method for identifying substances which prime cells for a stress response comprising the steps of:

   - inoculating cells in a suitable culture medium and culturing them in said medium;
   - adding one or more candidate substances to the culture medium thereby treating the cells with said candidate substance; and
   - measuring the production of at least one volatile organic compound in the cells treated with the candidate substance and in control cells not treated with the candidate substance,

   wherein an increase in the production of the at least one volatile organic compound in the cells treated with the candidate substance compared to the control cells indicates that the candidate substance primes the cells for a stress response.

2. Method according to claim 1, wherein the at least one volatile organic compound is selected from the group consisting of ethylene, nitric oxide and hydrogen peroxide.

3. Method according to claim 1 or 2, wherein the production of the at least one volatile substance is measured in a shaken measuring flask under sterile conditions, wherein during a purge phase air passes through the measuring flask and in a measuring phase the air stream is interrupted and the concentration of the at least one volatile organic compound is measured with a detecting element for said at least one volatile organic compound.

4. Method according to claim 3, further comprising calibrating the detecting element.

5. Method according to claim 4, wherein the detecting element is calibrated by passing a defined flow rate of the at least one volatile substance through the measuring flask during the purge phase and measuring the concentration of the at least one volatile substance with the detecting element.

6. Method for identifying substances which prime plant cells for a stress response comprising the steps of:

   - inoculating plant cells in a suitable culture medium and culturing them in said medium;
   - adding one or more candidate substances to the culture medium thereby treating the plant cells with said candidate substance; and

- measuring the production of ethylene in the cells treated with the candidate substance and in control cells not treated with the candidate substance,

wherein an increase in the production of ethylene in the cells treated with the candidate substance compared to the control cells indicates that the candidate substance primes the plant cells for a stress response.

**7.** Method according to claim 6, wherein the production of ethylene is measured in a shaken measuring flask under sterile conditions, wherein during a purge phase air passes through the measuring flask and in a measuring phase the air stream is interrupted and the concentration of ethylene is measured with a detecting element for ethylene.

**8.** Method according to any of the preceding claims, wherein the candidate substance is added 24 to 120 hours after inoculation.

**9.** Method according to any of the preceding claims, further comprising, after the addition of the candidate substance, subjecting the cells to a compound eliciting a stress response.

**10.** Method according to claim 9, wherein the cells are subjected to the compound eliciting a stress response 6 to 48 hours after the addition of the candidate substance.

**11.** Method according to any one of claims 2 to 10, wherein an increase in the production of ethylene is quantified using formula I:

$$Increase\ ETR = \frac{\int_{tx}^{ty} ETR_{experiment}}{\int_{tx}^{ty} ETR_{control}} - 1$$

wherein $\int_{tx}^{ty} ETR_{experiment}$ is the area under the curve for cells treated with the candidate substance and optionally with the compound eliciting a stress response, $\int_{tx}^{ty} ETR_{control}$ is the area under the curve for the control cells, wherein x is 1, 2 or 3 so that tx is the time point at which the candidate substance is added (t1) or the compound eliciting a stress response is added (t2) or at which the ethylene transfer rate has reached its minimum value after reaching the first maximum of the ethylene transfer rate (t3) and wherein y is 2, 3 or 4 so that ty is the time point at which the compound eliciting a stress response is added (t2) or at which the ethylene transfer rate has reached its minimum value after reaching the first maximum of the ethylene transfer rate (t3) or at which the ethylene transfer rate has reached its minimum value after reaching the second maximum of the ethylene transfer rate (t4), wherein y is greater than x.

**12.** Method according to any one of claims 2 to 10, wherein an increase in the production of ethylene is quantified using formula II:

$$\Delta ETR_1 = ETR_{+priming\ compound} - ETR_{w/o\ additives}$$

wherein $\Delta ETR_1$ is the difference in the ethylene transfer rate, $ETR_{+priming\ compound}$ is the ethylene transfer rate for cells treated with the candidate substance after 24 hours of treatment and $ETR_{w/o\ additives}$ is the ethylene transfer rate for untreated control cells treated at the same time point.

**13.** Device comprising at least one measuring flask arranged on a vibrator table, with an inlet and an outlet for a purge gas, a valve located at the inlet and a valve located at the outlet, at least one detecting element for measuring the concentration of at least one volatile organic compound in the measuring flask and a control computer for processing the electrical signals of each detecting device.

**14.** Device according to claim 13, wherein the detecting element is an ethylene or ethylene oxide sensor.

**15.** Use of the device according to claim 13 or 14 for the identification of substances which prime cells for a stress response.

Figure 1a

Figure 1b

Figure 2

Figure 3a

Figure 3b

Figure 4a

Figure 4b

Figure 4c

Figure 4d

Figure 4e

Figure 4f

Europäisches
Patentamt

European
Patent Office

Office européen
des brevets

# EUROPEAN SEARCH REPORT

Application Number

EP 16 15 8265

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/202463 A1 (RHEINISCH WESTFÄLISCHE TECH HOCHSCHULE AACHEN [DE]) 24 December 2014 (2014-12-24) | 7-12 | INV. G01N33/50 |
| Y | * abstract; claim 14 * | 6 | |
| X | WO 2012/029539 A1 (UNIV TOKYO SCI EDUC FOUND [JP]; KUCHITSU KAZUYUKI [JP]; KURUSU TAKAMIT) 8 March 2012 (2012-03-08) | 1-5,7-12 | |
| Y | * p 12, para 2; claims 1,8 * | 6 | |
| X | GEUN SONG ET AL: "Two Volatile Organic Compounds Trigger Plant Self-Defense against a Bacterial Pathogen and a Sucking Insect in Cucumber under Open Field Conditions", INTERNATIONAL JOURNAL OF MOLECULAR SCIENCES 2012 MDPI AG CHE, vol. 14, no. 5, 8 May 2013 (2013-05-08), pages 9803-9819, XP055274230, ISSN: 1661-6596, DOI: 10.3390/ijms14059803 | 7-12 | |
| Y | (p 9804, middle of para 1)(p 373, end of para 2)(p 9805, beginning of the last para); * abstract * | 6 | TECHNICAL FIELDS SEARCHED (IPC) G01N B01D |
| X | Marshall Digital Scholar ET AL: "Marshall University Ethylene production as an indicator of stress conditions in hydroponically-grown strawberries Recommended Citation", , 1 October 2008 (2008-10-01), XP055274594, Retrieved from the Internet: URL:http://mds.marshall.edu/cgi/viewcontent.cgi?article=1174&context=etd [retrieved on 2016-05-23] | 7-12 | |
| Y | * p 67, para 2 * | 6 | |

The present search report has been drawn up for all claims

| Place of search | Date of completion of the search | Examiner |
|---|---|---|
| Munich | 23 May 2016 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12

☐ The present supplementary European search report has been drawn up for those parts of the European patent application which relate to the invention first mentioned in the claims (Rule 164 (1) EPC).

**LACK OF UNITY OF INVENTION**
**SHEET B**

The Search Division considers that the present European patent application does not comply with the requirements of unity of invention and relates to several inventions or groups of inventions, namely:

1. claims: 1-12

   Method for identifying substances which prime cells for a stress response comprising measuring the production of at least one volatile organic compound in the cells treated with the candidate substance and in control cells not treated with the candidate substance, wherein an increase in the production of the at least one volatile organic compound in the cells treated with the candidate substance compared to the control cells indicates that the candidate substance primes the cells for a stress response.
   ---

2. claims: 13-15

   Device comprising at least one measuring flask arranged on a vibrator table, with an inlet and an outlet for a purge gas, a valve located at the inlet and a valve located at the outlet, at least one detecting element for measuring the concentration of at least one volatile organic compound in the measuring flask and a control computer for processing the electrical signals of each detecting device. Use of the device for the identification of substances which prime cells for a stress response.
   ---

## ANNEX TO THE EUROPEAN SEARCH REPORT
## ON EUROPEAN PATENT APPLICATION NO.

EP 16 15 8265

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2016

| Patent document cited in search report | | Publication date | Patent family member(s) | | Publication date |
|---|---|---|---|---|---|
| WO 2014202463 | A1 | 24-12-2014 | EP | 3010329 A1 | 27-04-2016 |
| | | | US | 2016130591 A1 | 12-05-2016 |
| | | | WO | 2014202463 A1 | 24-12-2014 |
| WO 2012029539 | A1 | 08-03-2012 | JP | 5885268 B2 | 15-03-2016 |
| | | | JP | 2013538039 A | 10-10-2013 |
| | | | US | 2015141252 A1 | 21-05-2015 |
| | | | WO | 2012029539 A1 | 08-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2015069593 W
- DE 4415444
- EP 0905229 A2

### Non-patent literature cited in the description

- **OERKE et al.** Crop production and crop protection. Elsevier Science B.V, 1994
- **CONRATH et al.** *Annual Review of Phytopathology,* 2015, vol. 53, 97-119
- **CONRATH.** *Trends in Plant Science,* 2011, vol. 16 (10), 524-531
- **CONRATH et al.** *Mol. Plant. Microbe Interact,* 2006, vol. 19 (10), 1062-1071
- **HAYES et al.** *J. Leukocyte Biol,* 1991, vol. 50, 176-181
- **HAYES et al.** *Blood,* 1995, vol. 86, 646-650
- **SIEGRIST et al.** *Physiol. Mol. Plant Pathol,* 1998, vol. 53 (4), 223-238
- **SCHILLING et al.** *BMC Plant Biology,* 2015, vol. 15, 282
- **HASE et al.** *Physiological and Molecular Plant Pathology,* 2003, vol. 62, 219-226
- **ANDERLEI ; BUECHS.** *Biochemical Engineering Journal,* 2001, vol. 7, 157-162
- **ANDERLEI et al.** *Biochemical Engineering Journal,* 2004, vol. 17, 187-194
- **NÜRNBERGER et al.** *Cell,* 1994, vol. 78, 449-460
- **FELLBRICH et al.** *Plant J.,* 2002, vol. 32 (3), 375-390
- **FELIX et al.** *Plant J,* 1999, vol. 18 (3), 265-276
- **ZIPFEL et al.** *Cell,* 2006, vol. 125, 749-760
- **VILLALBA-MATEOS et al.** *Mol. Plant Microbe Interact,* 1997, vol. 10, 1045-1053
- **CHALFOUN et al.** *J. Biol. Chem.,* 2013, vol. 288 (20), 14098-14113
- **ROTBLAT et al.** *Plant J.,* 2002, vol. 32 (6), 1049-1055